(19) Europäisches Patentamt
European Patent Office
Office européen des brevets

(11) Veröffentlichungsnummer: **0 193 885**
**B1**

(12) # EUROPÄISCHE PATENTSCHRIFT

(45) Veröffentlichungstag der Patentschrift: **14.06.89**

(51) Int. Cl.⁴: **C07D 333/38**, C07D 333/44, C07D 409/06, C09B 29/06

(21) Anmeldenummer: **86102603.7**

(22) Anmeldetag: **28.02.86**

(54) **Thiophenderivate.**

(30) Priorität: **02.03.85 DE 3507421**
**02.10.85 DE 3535134**

(43) Veröffentlichungstag der Anmeldung:
**10.09.86 Patentblatt 86/37**

(45) Bekanntmachung des Hinweises auf die Patenterteilung:
**14.06.89 Patentblatt 89/24**

(84) Benannte Vertragsstaaten:
**BE CH DE FR GB IT LI NL**

(56) Entgegenhaltungen:
**EP-A- 0 150 034**
**DE-A- 2 304 201**
**DE-A- 2 513 337**
**DE-A- 2 553 621**
**FR-A- 2 190 883**

**CHEMICAL ABSTRACTS, Band 104, Nr. 3, 20. Januar 1986, Seite 476, Nr. 19505f, Columbus, Ohio, US; & JP - A - 60 161 978 (MITSUI TOATSU CHEMICALS INC.) 23-08-1985**
**"The Chemistry of Heterocyclic Compounds - Thiophene and its Derivates", Interscience Publ. 1952**
**Z. Chem. 7, 186 (1967)**
**Chem. Ber. 101, 1933 (1968)**
**Chem. Ber. 98, 3571 (1965)**

(73) Patentinhaber: **BASF Aktiengesellschaft, Carl-Bosch-Strasse 38, D-6700 Ludwigshafen(DE)**

(72) Erfinder: **Schefczik, Ernst, Dr., Dubliner Strasse 7, D-6700 Ludwigshafen(DE)**
Erfinder: **Etzbach, Karl-Heinz, Dr., Bensheimer Ring 9 a, D-6710 Frankenthal(DE)**
Erfinder: **Ellingsfeld, Heinz, Dr., Pierstrasse 9 a, D-6710 Frankenthal(DE)**

(56) Entgegenhaltungen: (Fortsetzung)
**Z. Chem. 2, 305, (1962)**
**D.E. Wolf, K. Folkers "The Preparation of Thiophenes and Tetrahydrothiophenes", Org. React. 6, 410-468 (1951)**

**Die Akte enthält technische Angaben, die nach dem Eingang der Anmeldung eingereicht wurden und die nicht in dieser Patentschrift enthalten sind.**

EP 0 193 885 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Verbindungen der allgemeinen Formel I

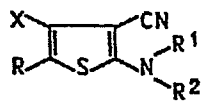

in der

X Fluor, Chlor, Brom, $SO_2Y$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_7$, $SCH_2C_6H_5$, $SC_2H_4OH$, $SCH_2COOCH_3$, $SCH_2COOC_2H_5$, $SC_6H_{11}$, $SC_6H_5$ oder $SC_6H_4CH_3$,

Y Alkyl, Alkenyl, Cycloalkyl, Aralkyl, Aryl, Chlor oder gegebenenfalls substituiertes Hydroxy oder Amino,

R Wasserstoff, $C_1$- bis $C_4$-Alkyl, Cl, Br. NO, $NO_2$, $SO_3H$, CHO, CN, $CH_3CO$, $C_2H_5CO$, $C_6H_5CO$, $CH_3SO_2$. $C_2H_5SO_2$, $C_6H_5SO_2$ oder ein Rest der Formel –CH=T, wobei T der Rest einer methylenaktiven Verbindung oder eines Amins ist,

$R_1$ Wasserstoff, Acyl oder gegebenenfalls substituiertes Alkyl, Cycloalkyl oer Alkenyl,

$R_2$ Wasserstoff oder gegebenenfalls substituiertes Alkyl oder Alkenyl,

$R_1$ und $R_2$ zusammen mit dem Stickstoff ein gesättigter Heterocyclus und

$R_1$ und $R_2$ zusammen ein Rest der Formel

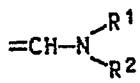

sind.

Reste Y sind beispielsweise $CH_3$, $C_2H_5$, $C_3H_7$, $C_4H_9$, $C_6H_{13}$, $C_6H_{11}$, $C_8H_{17}$,$C_6H_5$–$CH_2$, $C_6H_5$–$CH_2$–$CH_2$, $C_6H_5$, Cl–$C_6H_4$, $C_4H_9$–$C_6H_4$, Cl, OH, $CH_3O$, $C_2H_5O$, $C_3H_7O$, $C_4H_9O$, $C_6H_5$–$CH_2O$, $C_6H_5$–$CH_2O$, $C_6H_5$–$CH_2$–$CH_2O$, $C_6H_5O$, $ClC_6H_4O$, $CH_3C_6H_4O$, $NH_2$, $NHCH_3$, $N(CH_3)_2$, $NHC_2H_5$, $N(C_2H_5)_2$, $NHC_4H_9$, $N(C_4H_9)_2$, $NHC_6H_5$, $NHC_6H_5$, $NHC_6H_4$–$CH_3$, $NHC_6H_4Cl$ oder $NCH_3C_6H_4$.

Hethylenaktive Verbindungen der Formel $H_2T$ sind beispielsweise Verbindungen derFormel

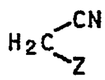

wobei Z Cyan, Nitro, Alkanoyl, Aroyl, Alkylsulfonyl, Arylsulfonyl, Carboxyl, Carbonester oder gegebenenfalls substituiertes Carbamoyl ist, sowie die Verbindungen der Formeln:

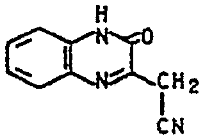
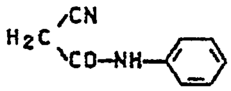
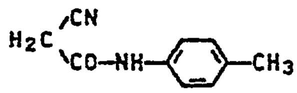
,
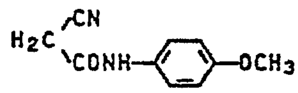

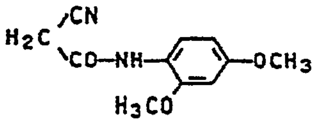
,
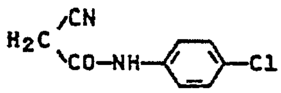
,
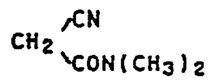
,
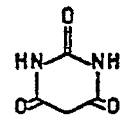

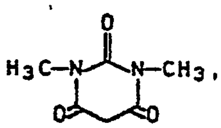
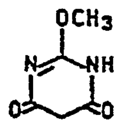
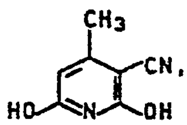
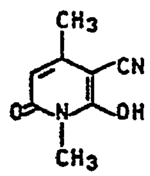
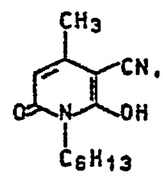

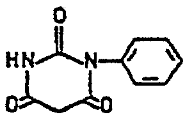
,
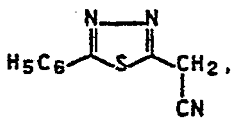
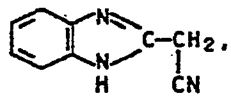
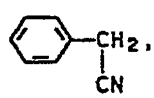
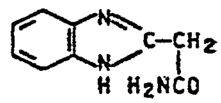

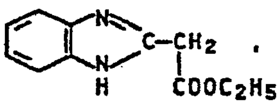
,
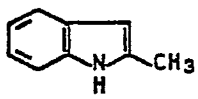
,
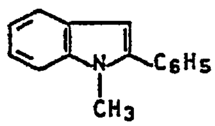
,
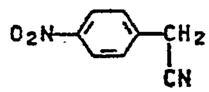

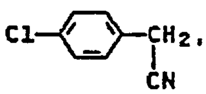
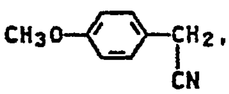
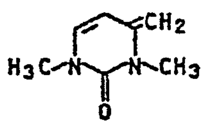
,

$CH_3-NO_2$, $C_2H_5-NO_2$,

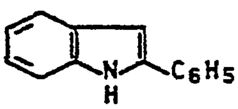
,
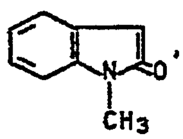
,
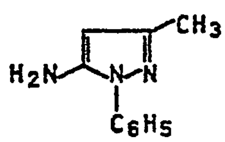
,
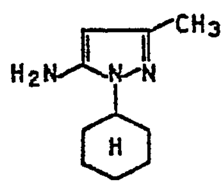
,

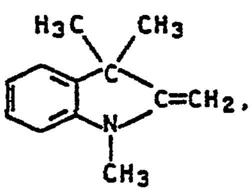
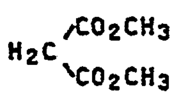
,
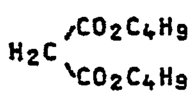
oder
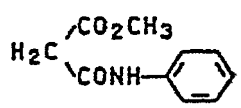

Einzelne wichtige Verbindungen der Formel

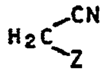 sind z.B.:

$H_2C(CN)_2$, 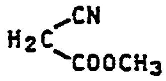, 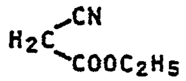, 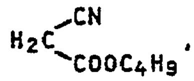, 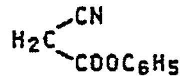,

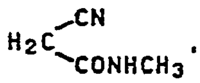, 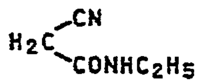, 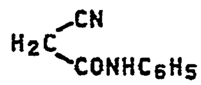, 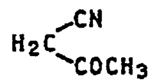 oder 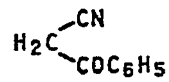

sowie 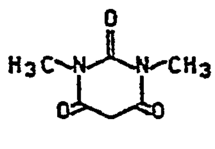, 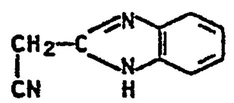, 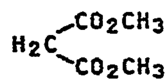, 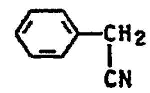

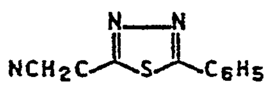 oder 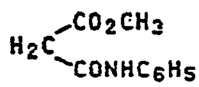.

Aminreste T sind z.B. $=N-C_6H_5$, $=N-C_6H_4CH_3$ oder allgemein Reste der Schiff' schen Basen der Amine.

Als Alkylreste für R sind z.B. $CH_3$, $C_2H_5$, $C_3H_7$ oder $C_4H_9$ zu nennen.

Reste $R^1$ und $R^2$ sind neben Wasserstoff im Rahmen der allgemeinen Definition z.B. gegebenenfalls durch Chlor, Brom, Cyan, Hydroxy, $C_1$- bis $C_4$- Alkoxy, $C_1$- bis $C_8$-Alkanoyloxy, $C_1$- bis $C_8$-Alkoxycarbonyl, Phenyl oder Tolyl substituiertes $C_1$- bis $C_4$-Alkyl, $C_3$-bis $C_5$-Alkenyl oder $C_5$- bis $C_7$-Cycloalkyl.

Im einzelnen seien beispielsweise genannt: $CH_3$, $C_2H_5$, $C_3H_7$, $C_4H_9$, $C_2H_4OH$, $CH_2CHOHCH_3$, $C_2H_4CN$, $C_2H_4OCH_3$, $C_2H_4OC_2H_5$, $C_2H_4OC_4H_9$, $C_2H_4OCOCH_3$, $C_2H_4OCOC_2H_5$, $C_2H_4OCOC_8H_{17}$, $C_2H_4COOCH_3$, $C_2H_4COOC_2H_5$, $C_2H_4COOC_4H_9$, $C_2H_4COOC_8H_{17}$, $CH_2C_6H_5$, $C_2H_4C_6H_5$, $CH_2C_6H_4CH_3$, $C_2H_4C_6H_4CH_3$, Cyclohexyl, Cycloheptyl, Allyl oder Methallyl.

Zusammen mit dem Stickstoff sind $R^1$ und $R^2$ z.B. Pyrrolidino, Piperidino, Morpholino, Piperazino oder N-Methylpiperazino.

Reste der Formel

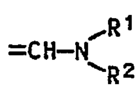

sind vorzugsweise

$=CH-N(CH_3)_2$, $=CH-N(C_2H_5)_2$ oder 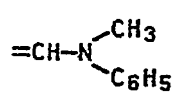

Zur Herstellung der Verbindungen der Formel I mit R = H oder $C_1$- bis $C_4$-Alkyl kann man Verbindungen der Formel II

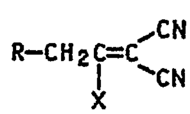

mit Schwefel abgebenden Verbindungen umsetzen. In die Verbindung der Formel I mit R = H können durch elektrophile Substitution Reste R nach den üblichen Methoden eingeführt werden.

Weiterhin kann die Verbindung der Formel I mit R = H und X = OH auch dadurch hergestellt werden, daß man die Verbindung der Formel

$ClCH_2COCl$

mit Malodinitril umsetzt und anschließend mit einem Sulfid reagieren läßt.

Einzelheiten der Herstellung können den Beispielen entnommen werden, in denen sich Angaben über Teile und Prozente, sofern nicht anders vermerkt, auf das Gewicht beziehen.

In der japanischen Offenlegungsschrift 84/42376 von Nippon Kayaku Co. ist angegeben ,daß man

durch Umsetzung von Mercaptoessigestern mit Malodinitril Verbindungen der Formel I erhalten würde. Wie jedoch schon aus J. Org. Chem. 38, 3616 (1973) sowie J. Heterocyclic Chem. 16, 1541 (1979) hervorgeht, trifft das nicht zu, denn bei diesen Reaktionen entstehen ausschließlich Thiazolderivate.

Die Verbindungen der Formel I eignen sich sowohl als Diazo- als auch als Kupplungskomponenten, sofern R = H ist.

Von besonderer Bedeutung als Diazokomponenten sind Verbindungen der Formel Ia

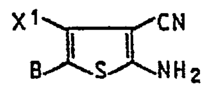

in der

$X^1$ Chlor, Hydroxy, $C_1$- bis $C_4$-Alkoxy oder -Alkylthio, Methylsulfonyl, Phenylsulfonyl, Hydroxysulfonyl, Phenoxy oder Phenylthio und

B Wasserstoff, $C_1$- bis $C_4$-Alkyl, Formyl, Acetyl, Nitro, Hydroxysulfonyl oder Cyan sind.

Weiterhin bevorzugt sind Verbindungen der Formel Ia, in der

$X^1$ Chlor, Methoxy, Ethoxy, Phenylthio, Methylsulfonyl oder Phenylsulfonyl und

B Formyl sind.

Weiterhin bevorzugt sind Verbindungen der Formel Ia, in der

$X^1$ Chlor, Methoxy, Ethoxy, Phenylthio, Methylsulfonyl oder Phenylsulfonyl und

B Cyan sind.

Weiterhin bevorzugt sind Verbindungen der Formel Ia, in der

$X^1$ Chlor, Methoxy, Ethoxy, Phenylthio, Methylsulfonyl oder Phenylsulfonyl und

B Nitro sind.

Weiterhin bevorzugt sind Verbindungen der Formel Ia, in der

$X^1$ Chlor, Methoxy, Ethoxy, Phenylthio, Methylsulfonyl oder Phenylsulfonyl und

B Hydroxysulfonyl sind.

Weiterhin bevorzugt sind Verbindungen der Formel Ia, in der

$X^1$ Chlor, Methoxy, Ethoxy, Phenylthio, Methylsulfonyl oder Phenylsulfonyl und

B ein Rest der Formel CH=T ist, wobei T

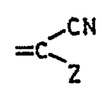

bedeutet und

Z Wasserstoff, Cyan, Carboxyl, Carbonester, gegebenenfalls substituiertes Carbamoyl, Benzimidazolyl, Benzoxazolyl oder Benzthiazolyl ist.

Als Kupplungskomponenten sind besonders wertvoll Verbindungen der Formel Ib

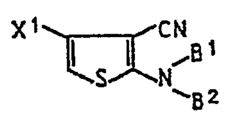

in der

$B^1$ Wasserstoff, $C_1$- bis $C_4$-Alkyl, $C_2$- oder $C_3$-Hydroxyalkyl, Cyanethyl, $C_1$- bis $C_4$-Alkoxycarbonylethyl, $C_1$- bis $C_4$- Alkanoyloxyethyl, Allyl, Benzyl, Phenylethyl oder Cyclohexyl und

$B^2$ Wasserstoff, $C_1$- bis $C_4$-Alkyl, $C_2$- oder $C_3$-Hydroxyalkyl, Cyanethyl,

$C_1$- bis $C_4$-Alkoxycarbonylethyl, $C_1$- bis $C_4$-Alkanoyloxyethyl oder Allyl sind und

$X^1$ die für Formel Ia angegebene Bedeutung hat.

Aus der FR-A 2 190 883 sind Azofarbstoffe bekannt, die Diazokomponenten auf Thiophenbasis aufweisen. Bei denjenigen Farbstoffen, deren Diazokomponenten unter die obengenannte Formel I fallen, waren diese Thiophenderivate aber nicht herstellbar.

Die DE-A 2 553 621 sowie die DE-A 2 304 201 beschreiben jeweils 2-Aminothiophene, die in Ringposition 4 entweder unsubstituiert oder durch Methyl, Phenyl oder Nitrophenyl substituiert sind.

Schließlich sind aus der EP-A 150 034 solche 2-Aminothiophene bekannt, die denen der Formel I ähnlich sind, jedoch in Ringposition 4 anstelle einer Hydroxysulfonylgruppe einen Carboxylrest aufweisen. Es wurde nun aber gefunden, daß ein mittels dieser Diazokomponente hergestellter Azofarbstoff im Vergleich zu einem Azofarbstoff, der mittels erfindungsgemäßen Diazokomponente hergestellt wurde, anwendungstechnische Mängel zeigt.

## Beispiele

### Beispiel 1

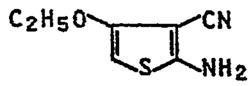

136 Teile 2-Cyan-3-ethoxicrotonsäurenitril werden in 200 Teilen N-Methylpyrrolidon gelöst und mit 32 Teilen Schwefelblüte versetzt. Nun gießt man 25 Teile Triethylamin zu. Dabei erwärmt sich das Reaktionsgemisch und der zunächst suspendierte Schwefel geht in Lösung. Bei Erreichen von 50°C wird mit einem Wasserbad gekühlt und die Temperatur auf 40–50°C gehalten. Nach 2 Stunden wird die klare Lösung mit 1000 Teilen Wasser versetzt, wobei das Reaktionsprodukt kristallin ausfällt. Es wird abgesaugt, mit Wasser gewaschen und bei 60°C im Vakuum getrocknet. Man erhält 142 Teile 2-Amino-3-cyan-4-ethoxithiophen in Form leicht braunstichiger Kristalle, die beim Lagern stark nachdunkeln. Eine aus Toluol umkristallisierte Probe zeigt einen Schmelzpunkt von 145–146°C und folgende Analysenwerte:
$C_7H_8N_2OS$ (168)
ber.: C 50,0 H 4,8 N 16,7 O 9,5 S 19,0
gef.: 50,0 4,9 16,8 9,8 18,9
IR- und NMR-Spektren stehen mit der Konstitution im Einklang.

### Beispiel 2

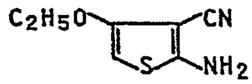

600 Teile Dimethylformamid, 40 Teile Triethylamin und 128 Teile Schwefelblüte werden bei Raumtemperatur gerührt. Nun gibt man anteilweise 544 Teile 2-Cyan-3-ethoxicrotonsäurenitril in dem Maße zu, daß die Temperatur des Reaktionsgemisches sich ohne Heizung bei 40 - 45 °C hält. Nach beendeter Zugabe rührt man 4 Stunden nach und versetzt dann mit 4000 Teilen Wasser. Man stellt die Kristallsuspension durch Zugabe von Essigsäure neutral und arbeitet wie in Beispiel 1 beschrieben auf.
Ausbeute: 623 Teile 2-Amino-3-cyan-4-ethoxyithiophen, das sind 92,7 % der Theorie.

### Beispiel 3

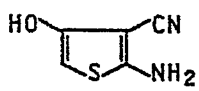

Zu einen siedenden Gemisch aus 168 Teilen 2-Amino-3-cyan-4-ethoxithiophen und 500 Raumteilen Methanol wird eine Lösung von 20 Raumteilen conc. Salzsäure in 100 Raumteilen Wasser getropft. Man kocht noch 2 Stunden unter Rückfluß, verdünnt mit 400 Raumteilen Wasser und saugt ab. Nach dem Waschen mit Wasser und Trocknen erhält man 136 Teile 2-Amino-3-cyan-4-hydroxithiophen. Eine aus Essigsäure umkristallisierte Probe schmilzt nicht bis 300 °C und zeigt folgende Analysenwerte:
$C_5H_4N_2OS$ (140)
ber.: C 42,9 H 2,9 N 20,0 O 11,4 S 22,9
gef.: 43,0 3,0 19,7 11,8 22,5
Das Produkt ist laut IR-Spektrum identisch mit der nach Beispiel 4 hergestellten Verbindung.

### Beispiel 4

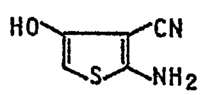

Zu einem Gemisch aus 178 Teilen Chloracetylochlorid, 104 Teilen Malonsäure dinitril und 900 Teilen Dimethylformamid werden unter Eiskühlung 350 Teile Triethylamin getropft. Man rührt die Lösung noch 1 h bei Raumtemperatur, gibt sie dann in eine Mischung aus 294,5 Teilen einer 40 %igen wäßrigen Ammoniumsulfidlösung, 1000 Teilen Eis und 1000 Teilen Wasser und rührt das Reaktionsgemisch noch 3 h bei Raumtemperatur. Der entstandene Niederschlag wird abgesaugt, mit Wasser gewaschen und getrocknet. Man erhält 137 Teile (62 % d. Th.) 2-Amino-3-cyan-4-hydroxy-thiophen.
Schmp.: > 300 °C (aus Eisessig), IR (KBr): 3260, 3061 ($NH_2$), 2219 (C≡N), 1668, 1641 $cm^{-1}$ (C=O).

Beispiel 5

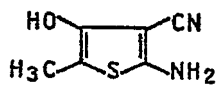

194 Teile Triethylorthopropionat und 66 Teile Malodinitril werden am absteigenden Kühler 1 h bei 100 °C gerührt. Zum Entfernen flüchtiger Bestandteile wird 30 Minuten lang Vakuum angelegt und dann erkalten gelassen. Man nimmt in 150 Raumteilen Dimethylformamid auf und gibt 32 Teile Schwefelblüte zu. Nun werden 100 Raumteile Triethylamin zugetropft, die Temperatur wird durch Kühlen bei < 60 °C gehalten. Man rührt noch 2 h bei 50 °C nach, setzt 500 Teile Wasser und 150 Teile conc. Salzäure zu und kocht 1 h. Nach dem Erkalten wird abgesaugt, mit Wasser gewaschen und getrocknet. Man erhält 131 Teile 2-Amino-3-cyan-4-hydroxi-5-methylthiophen. Die Verbindung ist alkalilöslich, eine aus Pentanol umkristallisierte Probe schmilzt bei 275 - 276 °C und zeigt folgende Analysenwerte:
$C_6H_8N_2OS$ (154)
ber.: C 46,8 H 3,9 N 18,2 O 10,4 S 20,8
gef.: 47,0 4,2 17,9 10,3 20,5

Beispiel 6

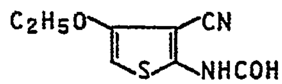

Zu 500 Teilen Acetanhydrid werden bei Raumtemperatur 200 Teile Ameisensäure getropft und nach 2 Stunden 168 Teile 2-Amino-3-cyan-4-ethoxithiophen eingetragen. Man rührt 4 h bei 50 °C und läßt dann 1000 Teile Wasser in der Wärme zulaufen. Nach dem Erkalten wird abgesaugt, mit Wasser gewaschen und getrocknet. Man erhält 174 Teile 2-Formylamino-3-cyan-4-ethoxithiophen von Schmelzpunkt 191 - 192 °C (aus Pentanol).

Beispiel 7

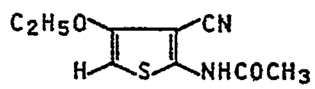

100 Teile wasserfreies Natriumacetat werden in 400 Raumteile Acetanhydrid eingetragen. Dazu gibt man 168 Teile 2-Amino-3-cyan-4-ethoxithiophen und kocht 4 h unter Rückfluß. Dann tropft man in der Wärme 800 Teile Wasser zu, läßt erkalten und saugt ab. Nach dem Waschen mit Wasser und Trocknen erhält man 194 Teile 2-Acetylamino-3-cyan-4-ethoxithiophen vom Schmelzpunkt 242 - 243 °C (aus Essigsäure).
$C_9H_{10}N_2O_2S$ (210)
ber.: C 51,4 H 4,8 N 13,3 O 15,2 S 15,3
gef.: 51,2 4,7 13,3 15,5 15,2
Analog wurde 2-Propionylamino-3-cyan-4-ethoxithiophen hergestellt, Schmelzpunkt 223 - 224 °C (aus Pentanol).

Beispiel 8

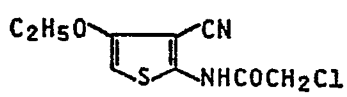

168 Teile 2-Amino-3-cyan-4-ethoxiothiophen werden in 500 Raumteilen Di methylformamid gelöst. Dazu gibt man 101 Teile Triethylamin und tropft bei 50 °C 130 Teile Monochloracetylchlorid zu. Man rührt 4 h bei 50 °C nach und trägt dann das Reaktionsgemisch auf 2000 Teile Wasser aus. Nach dem Absaugen, Waschen mit Wasser und Trocknen erhält man 240 Teile 2-(2-Chloracetylamino)-3-cyan-4-ethoxithiophen.

Eine aus Essigsäure umkristallisierte Probe schmilzt bei 243 - 244 °C und hat einen Chlorgehalt von 14,1 % (ber. 14,5 %).

Beispiel 9

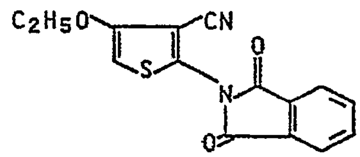

In 1000 Teilen Essigsäure werden 90 Teile wasserfreies Natriumacetat und 160 Teile Phthalsäureanhydrid gelöst. Dazu gibt man 168 Teile 2-Amino-3-cyan-4-ethoxithiophen und kocht 6 h unter Rückfluß. Man verdünnt mit 500 Teilen Wasser, läßt erkalten und saugt ab. Nach dem Waschen mit Wasser und Trocknen erhält man 203 Teile 2-Phthaloylimino-3-cyan-4-ethoxithiophen mit einem Schmelzpunkt von 173 - 174 °C (aus Essigsäure).

Beispiel 10

Zu einer Lösung von 200 Teilen Dimethylformamid in 2000 Raumteilen Toluol werden unter Kühlen bei 10 - 20 °C 350 Teile Phosphoroxitrichlorid zugetropft. Dann trägt man 168 Teile 2-Amino-3-cyan-4-ethoxithiophen ein und rührt 8 Stunden bei 40 °C. Nach dem Erkalten werden die abgeschiedenen Kristalle abgesaugt, mit Ethylacetat gewaschen und im Vakuum bei 30 °C getrocknet. Man erhält 271 Teile einer Verbindung der Konstitution

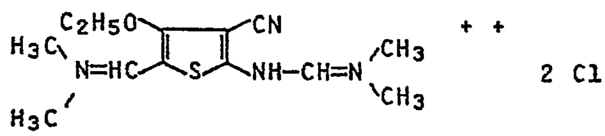

mit einem Cl⊖-Gehalt von 19,9 % (ber. 20,2 %), Schmelzpunkt 159 - 160 °C.

Beispiel 11

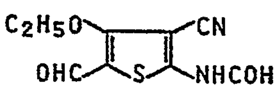

In ein Gemisch aus 1500 Raumteilen Chloroform und 250 Teilen Dimethylformamid werden unter Eiskühlung 400 Teile Phosphoroxitrichlorid eingetropft. Dann gibt man 168 Teile 2-Amino-3-cyan-4-ethoxithiophen zu und kocht 4 h unter Rückfluß. Beim Erkalten scheiden sich farblose Kristalle ab. Man verdünnt mit 500 Raumteilen Ethylacetat, saugt ab und wäscht die Kristalle mit Ethylacetat nach. Der Kristallkuchen wird in 2000 Teile Eiswasser eingetragen und unter Rühren mit Natronlauge auf pH =8 gestellt. Nach 4 Stunden wird abgesaugt, mit Wasser gewaschen und getrocknet. Man erhält 176 Teile 2-Formylamino-3-cyan-4-ethoxi-5-formylthiophen. Eine aus Pentanol umkristallisierte Probe schmilzt bei 230 - 231 °C und zeigt folgende Analysenwerte:
$C_9H_8N_2O_3S$ (224)
ber.: C 48,2 H 3,6 N 12,5 O 21,4 S 14,3
gef.: C 48,3 H 3,9 N 12,7 O 21,1 S 14,5

Beispiel 12

In 2000 Raumteile Methanol werden 224 Teile 1-Formylamino-2-cyan-3-ethoxi-4-formylthiophen und 120 Teile Hydroxylammoniumchlorid eingetragen und bei Raumtemperatur gerührt. Dazu gießt man eine Lösung von 80 Teilen Natriumacetat in 300 Teilen Wasser und kocht 6 Stunden unter Rückfluß. Nach dem Erkalten verdünnt man mit 1000 Teilen Wasser und saugt ab. Man erhält nach dem Trocknen 232 Teile einer Verbindung der Konstitution

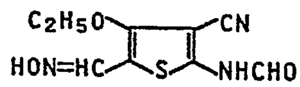

in Form farbloser Kristalle. Ein aus Pentanol umkristallisierte Probe schmilzt bei 255 - 256 °C und zeigt die folgenden Analysenwerte:
$C_9H_9N_3O_3S$ (239)
ber.: C 45,2 H 3,8 N 17,6 O 20,1 S 13,4
gef.: C 45,2 H 3,7 N 17,4 O 20,1 S 13,3

Beispiel 13

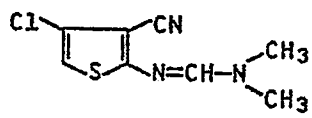

Zu 600 Teilen Dimethylformamid werden unter Eiskühlung 87,2 Teile Phosphoroxitrichlorid getropft. Man rührt das Gemisch 0,5 h bei 5 - 10°C, gibt dann 66,5 Teile 2-Amino-3-cyan-4-hydroxy-thiophen zu und erhitzt die Lösung 1 h auf 70 °C. Anschließend trägt man das Reaktionsgemisch in 2000 Teile Eiswasser ein, filtriert ab und versetzt das Filtrat unter Rühren mit 350 Teilen Natriumacetat. Das danach ausfallende Produkt wird abgesaugt, mit Wasser gewaschen und getrocknet. Man erhält 69,8 Teile (69 % d. Th.) N,N-Dimethyl-N'-(4-chlor-3-cyan-thienyl-2-)-formamidin.
Schmp.: 67 °C (aus Toluol/Hexan), IR, (KBr): 3090 (CH), 2222 (C≡N), 1636 $cm^{-1}$ (C=N).

Beispiel 14

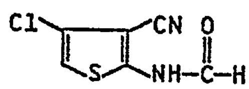

4,5 Teile N,N-Dimethyl-N'-(4-chloro-3-cyan-thienyl-2-)-formamidin werden in einer Mischung aus 20 Teilen Ameisensäure und 20 Teilen Wasser 1 h zum Sieden erhitzt. Nach dem Abkühlen auf Raumtemperatur wird das Produkt abgesaugt, mit Wasser gewaschen und getrocknet. Man erhält 3 Teile (77 % d. Th.) N-(4-chlor-3-cyan-thienyl-2-)-formamid.
Schmp.: 241 °C, IR (KBr): 2222 (C≡N), 1678, 1646 $cm^{-1}$ (C=O).

Beispiel 15

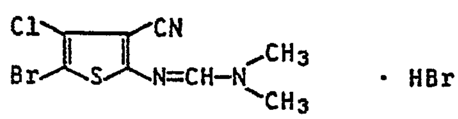

10,7 Teile N,N-Dimethyl-N'-(4-chlor-3-cyan-thienyl-2)-formamidin werden in 100 Teilen Eisessig gelöst, dann tropft man zu der Lösung 8 Teile Brom und erhitzt das Gemisch anschließend 3 h zum Sieden. Nach dem Abkühlen auf Raumtemperatur saugt man den entstandenen Niederschlag ab, wäscht ihn mit Eisessig, dann mit wäßriger Natriumbisulfitlösung und dann mit Wasser und trocknet ihn. Man erhält 13,4 Teile (72 % d. Th.) N,N-Dimethyl-N'-(5-brom-4-chlor-3-cyan-thienyl-2)-formamidin-hydrobromid.
Zers.-P.: 223 °C, IR (KBr): 2220 (C≡N), 1692, 1633 $cm^{-1}$ (C=N).

Beispiel 16

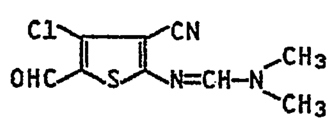

Zu 700 Teilen Dimethylformamid werden unter Eiskühlung 191 Teile Phosphoroxitrichlorid getropft, die

Mischung wird noch 0,5 h bei 5 - 10 °C gerührt, dann werden 70 Teile 2-Amino-3-cyan-4-hydroxy-thiophen eingetragen. Man rührt die Lösung noch 1 h bei 70 °C und gibt sie dann in 2000 Teile Wasser. Der entstandene Niederschlag wird abgesaugt, mit Wasser gewaschen und getrocknet. Man erhält 116 Teile (96 % d. Th.) N,N-Dimethyl-N'-(4-chlor-3-cyan-5-formyl-thienyl-2)-formamidin.

Schmp.: 186 °C (aus Toluol), IR (KBr): 2220 (C≡N), 1657, 1623 $cm^{-1}$ (C=O, C=N).

Beispiel 17

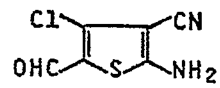

48,3 Teile N,N-Dimethyl-N'-(4-chlor-3-cyan-5-formyl-thienyl-2-)-formamidin werden in einer Mischung aus 200 Teilen Ameisensäure und 200 Teilen Wasser 3 h zum Sieden erhitzt. Nach dem Abkühlen auf Raumtemperatur wird das Produkt abgesaugt, mit Wasser gewaschen und getrocknet. Man erhält 33,5 Teile (90 % d. Th.) 2-Amino-4-chlor-3-cyan-5-formyl-thiophen.

Zers.-P.: 270 °C (aus Eisessig), IR (KBr): 3377, 3298, 3156 ($NH_2$), 2216 (C≡N), 1623 $cm^{-1}$ (C=O).

Beispiel 18

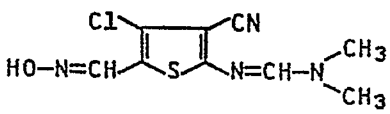

Man erhitzt eine Mischung aus 9,7 Teilen N,N-Dimethyl-N'-(4-chlor-3-cyan-5-formyl-thienyl-2-)formamidin, 2,8 Teilen Hydroxylamin-hydrochlorid, 3,3 Teilen Natriumacetat und 50 Teilen Dimethylformamid unter Rühren 3 h auf 50 °C. Anschließend gibt man die Lösung in 200 Teile Wasser, saugt den Niederschlag ab und trocknet ihn. Man erhält 8,5 Teile (83 % d. Th.) N,N-Dimethyl-N'-(4-chlor-3-cyan-5-(N-hydroxy-formimidyl-thienyl-2-)-formamidin

Schmp.: 199 °C (aus Eisessig), IR (KBr): 2220 (C≡N), 1639 $cm^{-1}$ (C=N).

Beispiel 19

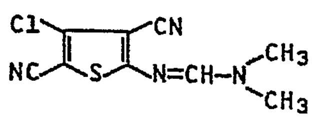

Zu 70 Teilen Dimethylformamid werden unter Eiskühlung 4,6 Teile Phosphoroxitrichlorid gegeben, anschließend rührt man die Mischung noch 0,5 h bei 5 - 10 °C und trägt dann 7,7 Teile N,N-Dimethyl-N'-(4-chlor-3-cyan-5-(N-hydroxy-formimidyl)-thienyl-2)-formamidin ein. Die Lösung wird noch 1 h bei Raumtemperatur gerührt und dann in 200 Teile Wasser gegeben. Der entstandene Niederschlag wird abgesaugt, mit Wasser gewaschen und getrocknet. Man erhält 6,1 Teile (85 % d. Th.) N,N-Dimethyl-N'-(4-chlor-3,5-dicyan-thienyl-2-)-formamidin.

Schmp.: 226 °C (aus Eisessig), IR, (KBr): 2235, 2225 (C≡N), 1628 $cm^{-1}$ (C=N).

Beispiel 20

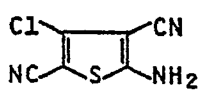

Eine Mischung aus 5,4 Teilen N,N-Dimethyl-N'-(4-chloro-3,5-dicyan-thienyl-2)-formamidin, 40 Teilen Ethanol und 4,5 Teilen konz. Salzsäure wird 2 h zum Sieden erhitzt, heiß filtriert und das Filtrat in 100 Teile Wasser gegeben. Nach dem Absaugen des ausgefallenen Niederschlags wird dieser mit Wasser gewaschen und getrocknet. Man erhält 3,8 g (92 % d. Th.) 2-Amino-4-chlor-3,5-dicyan-thiophen.

Schmp.: 259 °C (aus Eisessig), IR, (KBr): 3435, 3334, 3206, ($NH_2$), 2210 $cm^{-1}$ (C≡N).

Beispiel 21

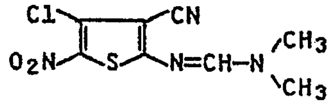

21,3 Teile N,N-Dimethyl-N'-(4-chlor-3-cyan-thienyl-2)-formamidin werden unter Eiskühlung in 100 Teile 100 %ige Salpetersäure eingetragen. Man läßt die Mischung 1 h bei Raumtemperatur rühren, fällt sie dann auf Eiswasser, saugt den entstandenen Niederschlag ab und wäscht ihn mit Wasser. Nach dem Trocknen erhält man 18,8 Teile (73 % d. Th.) N,N-Dimethyl-N'(4-chlor-3- cyan-5-nitro-thienyl-2)-formamidin.
Zers.-P.: 254 °C (aus Eisessig)

Beispiel 22

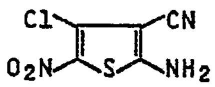

Eine Mischung aus 5,2 Teilen N,N-Dimethyl-N'-(4-chlor-3-cyan-5-nitro-thienyl-2-)-formamidin, 50 Teilen Ethanol und 5 Teilen konz. Salzsäure wird 3 h zum Sieden erhitzt und anschließend auf Eiswasser gegeben. Der Niederschlag wird abgesaugt, mit Wasser gewaschen und getrocknet. Man erhält 3,2 Teile (79 % d. Theorie) 2-Amino-4-chlor-3-cyan-5-nitro-thiophen.
Zers.-P.: 227 °C (aus o-Dichlorbenzol)

Beispiel 23

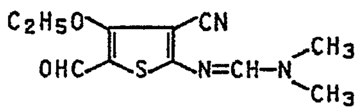

In 700 Teile Dimethylformanid wurden unter Eiskühlung 200 Teile Phosporoxirichlorid getropft. Danach ließ man 168 Teile 2-Amino-3-cyan-4-ethoxithiophen (Bsp.1) einlaufen und rührte 4 Stunden bei 40 °C nach. Nun wurden 100 Teile Methanol zugetropft und 200 Teile wasserfreies Natriumacetat eingetragen. Danach verrührte man das Reaktionsgemisch mit 100 Teilen Eis und tropfte 300 Teile 50 %ige Natriumhydroxidlösung zu. Man rührte über Nacht, saugte ab und wusch mit Wasser. Nach dem Trocknen erhielt man 181 Teile der Verbindung der angegebenen Konstitution mit einem Schmelzpunkt von 132 - 133 °C.
Analyse:
$C_{11}H_{13}N_3O_2S$ (251)
ber.: C 52,6 H 5,2 N 16,7 O 12,8 S 12,8
gef.: C 52,7 H 5,3 N 16,7 O 12,9 S 12,5

Beispiel 24

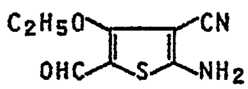

168 Teile 2-Amino-3-cyan-4-ethoxithiophen (Bsp. 1) werden in 500 Teilen Dimethylformanid gelöst. Dazu tropft man unter Rühren 200 Teile Phosphoroxitrichlorid. Die Reaktion ist extotherm, die Zutropfgeschwindigkeit wird so eingestellt, daß die Temperatur der Reaktionsmischung 50 ° nicht übersteigt. Man läßt 4 Stunden bei 50 ° nachrühren, setzt 50 Teile Eis zu und rührt, bis eine klare Lösung entstanden ist. Diese Lösung läßt man in 2000 Teile 12,5 %ige Natriumhydroxidlösung einlaufen und hält die Temperatur bei 20 bis 30°C Nach achtstündige Rühren bei Raumtemperatur wird abgesaugt, mit Wasser gewaschen und getrocknet. Man erhält 185 Teile 2-Amino-3-cyan-4-ethoxi-5-formylthiophen. Eine aus Pentanol umkristallisierte Probe schmilzt bei 245 - 246 ° und zeigt folgende Analysenwerte:
$C_8H_8N_2O_2S$ (196)
ber.: C 49,0 H 4,1 N 14,3 O 16,3 S 16,3
gef.: C 49,2 H 4,2 N 14,3 O 16,6 S 15,9

Beispiel 25

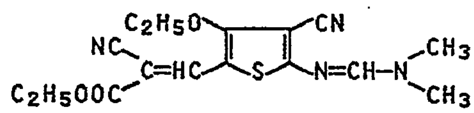

168 Teile 2-Amino-3-cyan-4-ethoxithiophen (Bsp. 1) werden in 750 Teilen Dimethylformamid gelöst und unter Kühlen bei 20 - 30 ° mit 200 Teilen Phosporoxitrichlorid versetzt. Nach 8 stündigem Rühren tropft man ein Gemisch von 120 Teilen Cyanessigsäureethylester und 250 Teilen absolutem Ethanol zu und setzt danach 400 Teile wasserfreies Natriumacetat zu. Man rührt über Nacht bei Raumtemperatur und verdünnt dann mit 1500 Teilen Wasser. Nach dem Absaugen, Waschen mit Wasser und Trocknen erhält man 294 Teile der Verbindung obiger Formel in form brauner Kristalle. Eine aus Ethanol umkristallisierte Probe schmilzt bei 153 - 154 ° und zeigt folgende Analysenwerte:
$C_{16}H_{18}N_4O_3S$ (346)
ber.: C 55,4 H 5,2 N 16,2 O 13,9 S 9,4
gef.: C 55,1 H 5,2 N 16,1 O 14,0 S 9,4

Ersetzt man den Cyanessigsäreethylester durch äquivalente Mengen der Methylenverbindungen 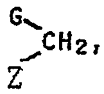 so erhält man bei gleicher Arbeitsweise die folgenden Verbindungen:

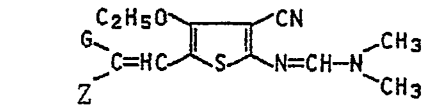

| G | Z | | Fp |
|---|---|---|---|
| —CN | —CN | braune Kristalle | 221-222° (Dimethylformanid) |
| —CN | $-COOCH_4H_9$ | gelbbraune Kristalle | 126-127° (Toluol) |
| —CN | 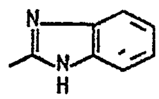 | orangerote Kristalle | > 350° (Dimethylformanid) |
| 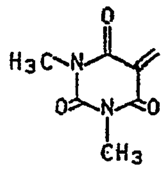 | | gelbbraune Kristalle | 293-294° (Dimethylformanid) |
| 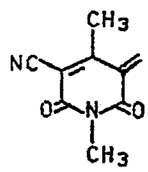 | | rotviolette Kristalle | 275-276° (Dimethylformanid) |
| 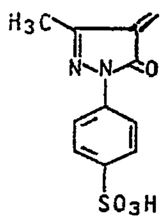 | | orangerot in Wasser löslich | > 350° |

Beispiel 26

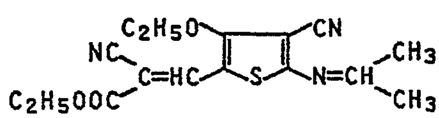

In 1500 Teile wasserfreies Ethanol werden 115 Teile Cyanessigsäureethylester und 251 Teile der nach Bsp.23 erhaltenen Verbindung eingetragen, dann wird 2 Stunden unter Rückfluß gekocht. Nach dem Erkalten wird abgesaugt und bei 50 °C getrocknet. Man erhält 278 Teile braune Kristalle, die Verbindung ist mit der nach Bsp. 25 erhaltenen identisch.

Beispiel 27

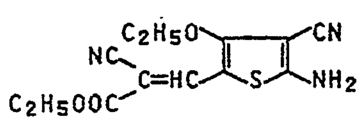

In ein Gemisch aus 500 Teilen N-Methylpyrroliden, 500 Teilen Wasser und 200 Teilen conc. Salzsäure werden 346 Teile der nach Bsp. 26 erhaltenen Verbindung eingetragen und dann wird 4 Stunden bei 100°C gerührt. Danach gibt man weitere 500 Teile Wasser zu, läßt erkalten und saugt ab. Nach dem Waschen mit Wasser und Trocknen erhält man 284 Teile einer gelben Verbindung;
Schmelzpunkt 222 - 223° (aus Essigsäure).

Analyse:
$C_{13}H_{13}N_3O_3S$ (291)
ber.: C 53,6 H 4,5 N 14,4 O 16,5 S 11,0
gef.: C 54,0 H 4,8 N 14,0 O 16,5 S 10,7

<u>Beispiel 28</u>

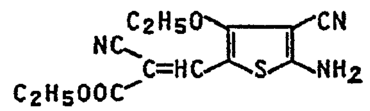

196 Teile 2-Amino-3-cyan-4-ethoxi-5-formylthiophen (Bsp. 24) und 125 Teile Cyanessigsäureethylester werden in 800 Teilen N-Mehtylpyrrolidon gelöst und mit 20 Teilen einer gesättigten wässrigen Natriumacetatlösung versetzt. Man rührt 12 Stunden bei 25 ° und verdünnt dann mit 2000 Teilen Wasser. Nach dem Absaugen und Trocknen erhält man 259 Teile der Verbindung, die nach IR-Spektren mit der nach Bsp. 27 hergestellten Verbindung identisch ist.

<u>Beispiel 29</u>

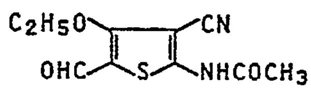

In 1000 Teile Dimethylformanid werden 210 Teile 2-Acetylamino-3-cyan-4-ethoxithiophen (Bsp. 7) eingetragen. Dazu läßt man innerhalb von 4 Stunden 200 Teile Phosphoroxitrochlorid tropfen. Nach 4 stündigem Nachrühren bei 60 ° bringt man den entstandenen Kristallbrei durch Zugabe von 600 Teilen Eis in Lösung. Diese Lösung wird in 2000 Teile 12,5 %ige Natriumhydroxidlösung eingerührt und nach 6 Stunden mit Salzsäure angesäuert. Das ausgefallene Produkt wird abgesaugt und mit Wasser gewaschen. Man erhält nach dem Trocknen 201 Teile 2-Acetylamino-3-cyan-4-ethoxi-5-formylthiophen. Eine aus Dimethylformanid umkristallisierte Probe zeigt einen Schmelzpunkt von 276-277 ° und folgende Analysenwerte:
$C_{10}H_{10}N_2O_3S$ (238)
ber.: C 50,4 H 4,2 N 11,8 O 20,1 S 13,5
gef.: C 50,5 H 4,3 N 11,9 O 20,4 S 13,2

<u>Beispiel 30</u>

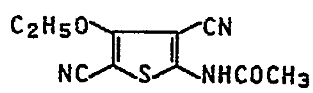

Ein Gemisch von 1000 Teilen Dimethylformanid, 1000 Teilen Wasser, 100 Teile Hydroxylammoniumchlorid, 100 Teilen Natriumacetat und 238 Teilen der nach Bsp. 29 erhaltenen Verbindung wird 6 Stunden bei 100 ° gerührt. Nach dem Erkalten verdünnt man mit weiteren 1000 Teilen Wasser, saugt ab und trocknet bei 100 °. Das getrocknete Produkt wird in 900 Teilen Acetanhy drid 4 Stunden unter Rückfluß gekocht, das Acetanhydrid dann durch Zutropfen von 60 Teilen Wasser zersetzt. Nach dem Erkalten wird abgesaugt, mit Wasser gewaschen und getrocknet. Man erhält 188 Teile 2-Acetylamino-3,5-dicyan-4-ethoxithiophen in Form graustichiger Kristalle.  Eine Aus Dimethylformanid umkristallisierte Probe hat einen Schmelzpunkt von 267-268 °C und folgende Analyse:
$C_{10}H_9N_3O_2S$ (235)
ger.: C 51,1 H 3,8 N 17,9 O 13,6 S 13,6
gef.: C 51, 0 H 3,7 N 17,6 O 14,0 S 13,4

Beispiel 31

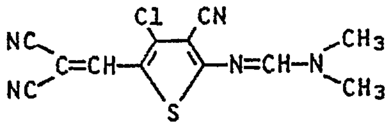

54,3 Teile N,N-Dimethyl-N'-(4-chlor-3-cyan-5-formylthienyl-2-)-formamidin, 14,9 Teile Malonsäuredinitril, 1 Teil ß-Alanin und 200 Teile Dimethylformamid werden 1 h auf 100 °C erhitzt. Nach dem Abkühlen auf Raumtemp. saugt man das ausgefallene orangefarbene Produkt ab, wäscht es mit Dimethylformamid und dann mit Wasser und trocknet es. Man erhält 50 Teile (77% d.Th.) einer goldgelben Verbindung der obigen Konstitution.

Schmp: 257 °C (aus DMF), IR (KBr): 2230, 2218 (C≡N), 1625, 1569 $cm^{-1}$ (C=C, C=N).
$\lambda_{max}(CH_2Cl_2)$: 458 nm, ε : 40 800
$C_{12}H_8ClN_5S$ (289,5)
ber.: C 49,7 H 2,8 Cl 12,3 N 24,2 S 11,1
gef.: C 49,6 H 2,7 Cl 12,4 N 24,2 S 11,0

Beispiel 32

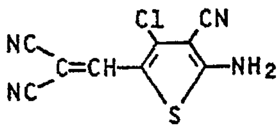

56 Teile 2-Amino-4-chlor-3-cyan-5-formylthiophen, 19,8 Teile Malonsäuredinitril, 1 Teil ß-Alanin und 200 Teile Dimethylformamid werden 3 h auf 100 °C erhitzt, anschließend gibt man die Reaktionsmischung auf Eiswasser. Der gelbe Niederschlag wird abgesaugt, mit Wasser gewaschen und getrocknet. Man erhält 65,3 Teile (93 % d. Th.) der obigen Verbindung.

Schmp: > 300 °C

Beispiel 33

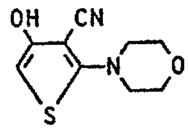

Man legt 26 Teile Chloracetylchlorid und 39, 1 Teile Cyanessigsärethiomorpholid in 140 Teilen Dimethylformamid vor und tropft under Eiskühlung 46,5 Teile Triethylamin zu. Nach einstündigem Rühren bei Raumtemperatur fällt man die Lösung auf Wasser, saugt den Niederschlag ab, wäscht ihn mit Wasser und trocknet ihn. Man erhält 30 Teile (62 % d. Th.) 3-Cyan-4-hydroxi-2-morpholinothiophen.

Schmp: 191 °C (Aus Eisessig), IR (KBr): 2204 (C≡N), 1649 (C=O), 1562 $cm^{-1}$ (C=C).
$C_9H_{10}N_2O_2S$ (210)
Ber.: C 51,4 H 4,8 N 13,3 O 15,2 S 15,3
Gef.: C 51,6 H 4,9 N 13,3 O 15,3 S 15,1

Beispiel 34

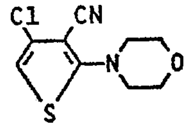

Zu einer Mischung aus 10,5 Teilen 3-Cyan-4-hydroxi-2-morpholino-thiophen, 5,1 Teilen Triethylamin und 50 Teilen Tetrahydrofuran tropft man 77 Teile Phosphoroxitrochlorid und erhitzt anschließend 1 h zum Sieden. Dann fällt man den Ansatz auf Wasser, saugt den Niederschlag ab, wäscht ihn mit Wasser und trocknet ihn. Man erhält 9,7 Teile (85 % d.Th.) 4-Chlor-3-cyan-2-morpholino-thiophen.

Schmp: 128 °C (aus iso-Propanol), IR, (KBr): 3109 (CH), 2212 $cm^{-1}$ (C≡N).
$C_9H_9ClN_2S$ (228,5)

Ber.: C 47,3 H 4,0 Cl 15,5 N 12,3 O 07,0 S 14,0
Gef.: C 47,0 H 4,0 Cl 15,3 N 12,0 O 7,4 S 13,9

Beispiel 35

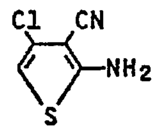

Zu einer Mischung aus 14 Teilen 2-Amino-3-cyan-4-hydroxithiophen, 7,9 Teilen Pyridin und 70 Teilen Tetrahydrofuran gibt man 15,3 Teile Phosphoroxitrichlorid und erhitzt anschließend 1 h zum Sieden. Nach dem Abkühlen gibt man den Ansatz auf Wasser, läßt über Nacht stehen, saugt den Niederschlag ab, wäscht ihn mit Wasser und trocknet ihn. Man erhält 9,4 Teile (60 % d.Th) 2-Amino-4-chlor-3-cyan-thiophen.

Schmp: 230 °C (aus Eisessig), IR (KBr): 3418, 3329, 3210, ($NH_2$), 3118 (CH), 2215 (C≡N), 1627 $cm^{-1}$
$C_5H_3ClN_2S$ (158,5)
Ber.: C 37,9 H 1,9 Cl 22,4 N 17,7 S 20,2
Gef.: C 37,9 H 1,8 Cl 22,1 N 17,2 S 20,8

Beispiel 36

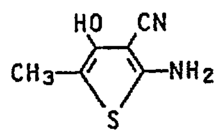

Die Herstellung der Verbindung erfolgt wie in Beispiel 4 beschrieben, anstelle von Chloracetylchlorid wird α-Chloropropionylchlorid eingesetzt. Man erhält in 50 %iger Ausbeute das 2-Amino-3-cyan-4-hydroxi-5-methyl-thiophen, das laut Schmelzpunkt und IR-Spektren mit der nach Beispiel 3 hergestellten Verbindung identisch ist.

Beispiel 37

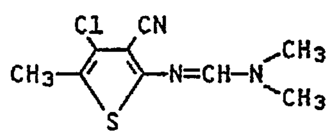

Wie in Beispiel 16 beschrieben, erhält man aus 15 Teilen Phosphorixitrichlorid, 100 Teilen Dimethylformamid und 12,3 Teilen 2-Amino-3-cyan-4-hydroxi-5-methyl-thiophen 14 Teile (77 % d.Th.) N,N-Dimethyl-N'-(4-chlor-3-cyan-5-mehtylthienyl-2-)-formamidin.

Schmp: 84 °C, IR (KBr): 2216 (C≡N), 1622 $cm^{-1}$ (C=N)
$C_9H_{10}ClN_2S$ (227,5)
Ber.: C 47,5 H 4,4 Cl 15,6 N 18,5 S 14,1
Gef.: C 47,5 H 4,4 Cl 15,7 N 18,4 S 14,0

Beispiel 38

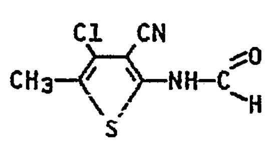

6,8 Teile N,N-Dimethyl-N'-(4-chlor-3-cyan-5-methyl-thienyl-2)-formamidin, 30 Teile Ameisensäure und 30 Teile Wasser werden 1 h zum Sieden erhitzt, das ausgefallene Produkt wird kalt abgesaugt, mit Wasser gewaschen und getrocknet. Man erhält 5,6 Teile (93 % d.Th) N-(4-chlor-3-cyan-5-methyl-thienyl-2)-formamid.

Schmp: 258 °C, IR (KBr): 3170 (NH), 2224 (C≡N), 1685, 1644, 1580 $cm^{-1}$ (C=O)
$C_7H_5ClN_2OS$ (200,5)

Ber.: C 41,9 H 2,5 Cl 17,7 N 14,0 O 8,0 S 16,0
Gef.: C 42,0 H 2,6 Cl 17,8 N 13,9 O 8,4 S 15,8

Beispiel 39

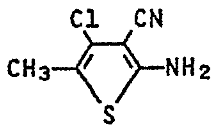

Eine Mischung aus 6,1 Teilen N,N-Dimethyl-N'-(4-chlor-3-cyan-5-methyl-thienyl-2)formamidin, 50 Teilen Ethanol und 2 Teilen konz. Salzsäure wird 1 h zum Sieden erhitzt. Anschließend gibt man das Gemisch in eine verdünnte wässrige Natriumacetatlösung, saugt den Niederschlag ab und trocknet ihn. Man erhält 4 Teile (86 % d.Th) 2-Amino-4-chlor-3-cyan-5-methyl-thiophen.

Schmp:158 °C, IR (KBr): 3420, 3323 ($-NH_2$), 2214, $cm^{-1}$ (C≡N).
$C_6H_5ClNS$ (172,5)
Ber.: C 41,7 H 2,9 Cl 20,5 N 16,2 S 18,6
Gef.: C 42,0 H 3,1 Cl 20,0 N 16,4 S 18,0

Beispiel 40

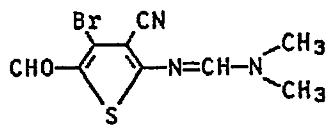

Zu 700 Teilen Dimethylformamid werden unter Eiskühlung 400 Teile Phosphoroxitribromid gegeben, die Mischung wird noch 0,5 h bei 5 - 10 °C gerührt, dann werden 78 Teile 2-Amino-3-cyan-4-hydroxi-thiophen eingetragen. Man rührt die Lösung 1 h bei 70 °C nach und gibt sie dann in 2000 Teile Eis wasser. Der Niederschlag wird abgesaugt, mit Wasser gewaschen und getrocknet. Man erhält 94 Teile (59 % d.Th) N,N-Dimethyl-N'-(4-brom-3-cyan-5-formyl-thienyl-2)-formamidin.

Schmp: 220 °C (aus Eisessig), IR, (KBr): 2222 (C≡N), 1620 $cm^{-1}$ (C=O):
$C_9H_88rN_3OS$ (286)
Ber.: C 37,8 H 2,8 Br 27,9 N 14,7 O 5,6 S 11,2
Gef.: C 37,9 H 3,3 Br 27,5 N 14,6 O 6,0 S 11,1

Beispiel 41

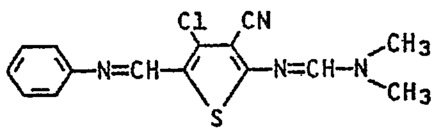

Eine Mischung aus 12,1 Teilen N,N-Dimethyl-N'-(4-chlor-3-cyan-5-formyl-thienyl-2-)-formamidin, 9,3 Teilen Anilin und 100 Teilen Methylglykol wird 1 h zum Sieden erhitzt. Dann wird der Niederschlag kalt abgesaugt, mit Methylgylkol und anschließend mit Wasser gewaschen und getrocknet.

Ausbeute: 11,9 Teile (75 % d.Th) N,N-Dimethyl-N'-(4-chlor-3-cyan-5-phenyl-iminomethyl-thienyl-2)-formamidin.

Schmp: 174 °C (aus Methylglykol), IR (KBr): 2220 (C≡N), 1634 $cm^{-1}$ (C=N)
$C_{15}H_{13}ClN_4S$ (316,5)
Ber.: C 56,9 H 4,1 Cl11,2 N 17,7 S 10,1
Gef.: C 56,8 H 4,1 Cl 11,2 N 17,4 S 10,0

Beispiel 42

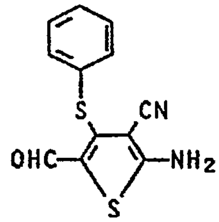

Zu einer Lösung aus 11 Teilen Thiophenol, 5,4 Teilen einer 30 %igen methanolischen Natriummethylatlösung und 100 Teilen Methanol gibt man 18,7 Teile 2-Amino-4-chlor-3-cyan-5-formylthiophen. Die Mischung wird 0,5 h zum Sieden erhitzt, dann läßt man abkühlen, saugt das ausgefallene Produkt ab, wäscht es mit Methanol und dann mit Wasser und trocknet es. Man erhält 21 Teile (81 % d.Th) 2-Amino-3-cyan-5-formyl-4-phenylthio-triophen.

Schmp: 230 °C (aus Eisessig), IR (KBr): 3360, 3292, 3142 ($NH_2$), 2220 (C≡N), 1643, 1621, 1588 $cm^{-1}$ (C=O, C=C).

$C_{12}H_8N_2OS_2$ (260)

Ber.: C 55,4 H 3,1 N 10,8 O 6,2 S 24,6

Gef.: C 55,3 H 3,2 N 10,7 O 6,6 S 24,0

Beispiel 43

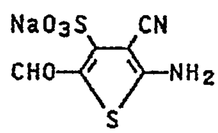

Man erhitzt 93,3 Teile 2-Amino-4-chlor-3-cyan-5-formyl-thiophen, 126 Teile Natriumsulfit und 300 Teile Wasser 2 h zum Sieden, gibt dann unter Eiskühlung 200 Teile konz. Salzsäure zu und läßt die Mischung über Nacht stehen. Dann saugt man ab, wäscht den Rückstand mit wenig Eiswasser und trocknet ihn. Man erhält 85 Teile (67 %d. Th) Natrium-(2-amino-3-cyan-5-formyl-thienyl-4)-sulfonat.

Schmp: > 300 °C, IR (KBr): 3385, 3303, 3191 ($NH_2$), 2233 (C≡N), 1608 $cm^{-1}$ (C=O)

$C_6H_3N_2NaO_4S$ (254)

Ber.: C 28,4 H 1,2 N 11,0 Na 9,1 O 25,2 S 25,2

Gef.: C 28,3 H 1,2 N 10,9 Na 8,7 O25,7 S 24,7

Beispiel 44

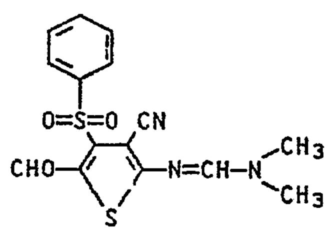

Eine Mischung aus 12,1 Teilen N,N-Dimethyl-N"-(4-chlor-3-cyan-5-formyl-thienyl-2)-formamidin, 8,8 Teilen Natriumphenylsulfinat und 70 Teilen Methylglykol wird 1 h zum Sieden erhitzt. Das ausgefallene Produkt wird dann kalt abgesaugt, mit Methylglykol und dann Wasser gewaschen und getrocknet. Man erhält 9,7 Teile, N,N-Dimethyl-N-(3-cyan-5-formyl-4-phenylsulfonyl-thienyl-2)-formamidin.

Schmp: 262 °C, IR (KBr): 2225 (C≡N), 1649, 1622 cm⁻ (C=N, C=O).

$C_{15}H_{13}N_3O_3S$ (347)

Ber.: C 51,9 H 3,8 N 12,1 O 13,8 S 18,5

Gef.: C 52,0 H 3,9 N 12,1 O 14,0 S 18,6

Beispiel 45

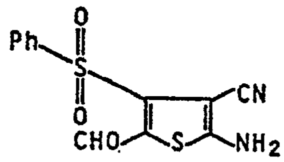

Zu einer Mischung aus 93,3 g 2-Amino-4-chlor-3-cyan-5-formyl-thiophen in 500 Teilen Methylglykol wird eine Lösung von 82 Teilen Natriumphenylsulfinat in 500 Teilen Wasser gegeben und das Reaktonsgemisch 6h zum Sieden erhitzt. Anschließend wird die Lösung heiß filtriert und das Filtrat über Nacht stehen gelassen. Das ausgefallene Produkt wird dann abgesaugt mit Wasser gewaschen und getrocknet. Man erhält 121 Teile (83 % d.Th.) 2-Amino-3-cyan-5-formyl-4-phenylsulfonyl-thiophen.
Zers.-P.: 230°C (aus, Eisessig), JR (KB.): 3316, 3217 ($NH_2$), 2220 (CN), 1648, 1626, 1609 $cm^{-1}$.
Die erfindungsgemäßen Verbindungen der Formel

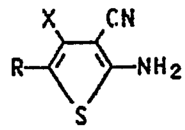

eignen sich als Diazokomponenten und können beispielsweise in Eisessig/Propionsäuremischungen oder in Schwefelsäure mit Nitrosylschwefelsäure diazotiert und anschließend mit Kupplungskomponenten umgesetzt werden.

Beispiel 46

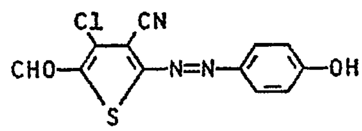

9,3 Teile 2-Amino-4-chlor-3-cyan-5-formylthiophen werden bei maximal 20 °C in 60 Teilen konz. Schwefelsäure gelöst, dann werden zu dieser Lösung bei 0 - 5 °C 16,6 Teile Nitrosylschwefelsäure (11,5 % $N_2O_3$) getropft. Man rührt noch 4 h bei 0 - 5 °C und läßt die so erhaltene Diazoniumsalzlösung bei 0 °C in eine Mischung aus 4,7 Teilen Phenol, 3 Teilen Natriumhydroxid, 0,5 Teilen Amidosulfonsäure, 200 Teilen Wasser und 400 Teilen Eis langsam einlaufen. Nach beendeter Kupplung wird der Farbstoff neutral gewaschen und getrocknet. Man erhält 12,2 Teile (84 % d.Th) des gelben Farbstoffs der obigen Formel.
$\lambda_{max}(CH_2Cl_2)$: 438 nm, ε: 24200

## Patentansprüche

1. Thiophenderivate der allgemeinen Formel I

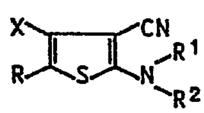

in der
X Fluor, Chlor, Brom, $SO_2Y$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H_5$, $SC_2H_4OH$, $SCH_2COOCH_3$, $SCH_2COOC_2H_5$, $SC_6H_{11}$, $SC_6H_5$ oder $SC_6H_4CH_3$,
Y Alkyl, Alkenyl, Cycloalkyl, Aralkyl, Aryl, Chlor oder gegebenenfalls substituiertes Hydroxy oder Amino,
R Wasserstoff, C1- bis C4-Alkyl, Cl, Br, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$ oder ein Rest der Formel –CH=T, wobei T der Rest einer methylenaktiven Verbindung oder eines Amins ist,
$R^1$ Wasserstoff, Acyl oder gegebenenfalls substituiertes Alkyl, Cycloalkyl oder Alkenyl,
$R^2$ Wasserstoff oder gegebenenfalls substituiertes Alkyl ode Alkenyl,

$R^1$ und $R^2$ zusammen mit dem Stickstoff ein gesättigter Heterocyclus und
$R^1$ und $R^2$ zusammen ein Rest der Formel

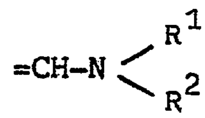

sind.

2. Verbindungen gemäß Anspruch 1 der Formel

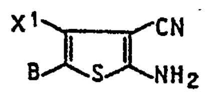

in der
$X^1$ Chlor, Hydroxy, $C_1$- bis $C_4$-Alkoxy oder -Alkylthio, Methysulfonyl, Phenylsulfonyl, Hydroxysulfonyl, Phenoxy oder Phenylthio und
B Wasserstoff, $C_1$- bis $C_4$-Alkyl, Formyl, Acetyl, Nitro, Hydroxysulfonyl oder Cyan sind.

3. Verbindungen gemäß der Formel in Anspruch 2, wobei
$X^1$ Chlor, Methoxy, Ethoxy, Phenylthio, Methylsulfonyl oder Phenylsulfonyl und
B Formyl sind.

4. Verbindungen gemäß der Formel in Anspruch 2, wobei
$X^1$ Chlor, Methoxy, Ethoxy, Phenylthio, Methylsulfonyl oder Phenylsulfonyl und
B Cyan sind.

5. Verbindungen gemäß der Formel in Anspruch 2, wobei
$X^1$ Chlor, Methoxy, Ethoxy, Phenylthio, Methylsulfonyl oder Phenylsulfonyl und
B Nitro sind.

6. Verbindungen gemäß der Formel in Anspruch 2, wobei
$X^1$ Chlor, Methoxy, Ethoxy, Phenylthio, Methylsulfonyl oder Phenylsulfonyl und
B Hydroxysulfonyl sind

7. Verbindungen gemäß der Formel in Anspruch 2, wobei
$X^1$ Chlor, Methoxy, Ethoxy, Phenylthio, Methylsulfonyl oder Phenylsulfonyl und
B ein Rest der Formel CH=T ist, wobei T

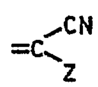

bedeutet und
Z Wasserstoff, Cyan, Carboxyl, Carbonester, gegebenenfalls substituiertes Carbamoyl, Benzimidazolyl, Benzoxazolyl oder Benzthiazolyl ist.

8. Verbindungen gemäß Anspruch 1 der Formel

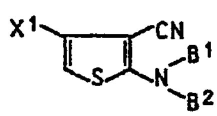

in der
$B^1$ Wasserstoff, $C_1$- bis $C_4$-Alkyl, $C_2$- oder $C_3$-Hydroxyalkyl, Cyanethyl, $C_1$- bis $C_4$-Alkoxycarbonylethyl, $C_1$- bis $C_4$-Alkanoyloxyethyl, Allyl, Benzyl, Phenylethyl oder Cyclohexyl,
$B^2$ Wasserstoff, $C_1$- bis $C_4$-Alkyl, $C_2$- oder $C_3$-Hydroxyalkyl, Cyanethyl, $C_1$- bis $C_4$-Alkoxycarbonylethyl, $C_1$- bis $C_4$-Alkanoyloxyethyl oder Allyl oder $B^1$ und $B^2$ zusammen mit dem Stickstoff Pyrrolidino, Piperidino, Morpholino, Piperazino, N-Methylpiperazino oder N-ß-hydroxyethylpiperazino sind und
$X^1$ die für Anspruch 2 angegebene Bedeutung hat.

9. Verwendung der Verbindungen gemäß Anspruch 1 als Diazo- oder Kupplungskomponenten.

10. Verfahren zur Herstellung der Verbindung der Formel

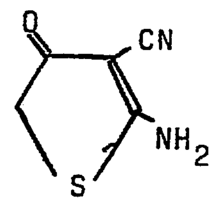

dadurch gekennzeichnet, daß man Malonsäuredinitril mit Chloracetylchlorid umsetzt und anschließend mit einem Sulfid reagieren läßt.

11. Verfahren gemäß Anspruch 10, dadurch gekennzeichnet, daß man die Umsetzung in einem Lösungsmittel vornimmt.

12. Verfahren gemäß Anspruch 10, dadurch gekennzeichnet, daß man eine Base zusetzt.

13. Verfahren zur Herstellung der Verbindung der Formel

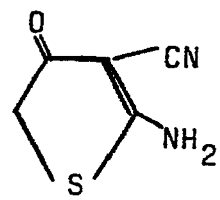

dadurch gekennzeichnet, daß man 2-Cyan-3-alkoxycrotonsäurenitril mit Schwefel abgebenden Verbindungen umsetzt und das Produkt entalkyliert.

14. Verfahren gemäß Anpsruch 13, dadurch gekennzeichnet, daß man die Umsetzung in einem Lösungsmittel vornimmt.

15. Verfahren gemäß Anspruch 13, dadurch gekennzeichnet, daß man eine Base zusetzt.

## Claims

1. A thiphene derivative of the formula I

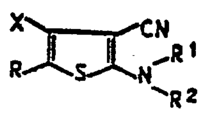

where

X is fluorine, chlorine, bromine, $SO_2Y$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H$, $SC_2H_4OH$, $SCH_2COOCH_3$, $SCH_2COOC_2H_5$, $SC_6H_{11}$, $SC_6H_5$ oder $SC_6H_4CH_3$,

Y is alkyl, alkenyl, cycloalkyl, aralkyl, aryl, chlorine or unsubstituted or substituted hydroxyl or amino,

R is hydrogen, C1–C4-alkyl, Cl, Br, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$ or a radical of the formula –CH=T in which T is a radical of a methylene-active compound or of an amine, and

$R^1$ is hydrogen, acyl or unsubstituted or substituted alkyl, cycloalkyl or alkenyl and

$R^2$ is hydrogen or unsubstituted or substituted alkyl or alkenyl, or

$R^1$ and $R^2$ together with the nitrogen form a saturated heterocyclic structure, and

$R^1$ and $R^2$ together form a radical of the formula

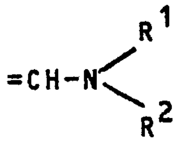

2. A compound as claimed in claim 1, of the formula

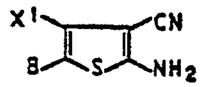

where

$X^1$ is chlorine, hydroxyl, $C_1$–$C_4$-alkoxy, $C_1$–$C_4$-alkylthio, methylsulfonyl, phenylsulfonyl, hydroxysulfonyl, phenoxy or phenylthio and

B is hydrogen, $C_1$–$C_4$-alkyl, formyl, acetyl, nitro, hydroxysulfonyl or cyano.

3. A compound of the formula in claim 2, wherein
$X^1$ is chlorine, methoxy, ethoxy, phenylthio, methylsulfonyl or phenylsulfonyl and
B is formyl.

4. A compound of the formula in claim 2, wherein
$X^1$ is chlorine, methoxy, ethoxy, phenylthio, methylsulfonyl or phenylsulfonyl and
B is cyano.

5. A compound of the formula in claim 2, wherein
$X^1$ is chlorine, methoxy, ethoxy, phenylthio, methysulfonyl or phenylsulfonyl and
B is nitro.

6. A compound of the formula in claim 2, wherein
$X^1$ is chlorine, methoxy, ethoxy, phenylthio, methylsulfonyl or phenylsulfonyl and
B is hydroxysulfonyl.

7. A compound of the formula in claim 2, wherein
$X^1$ is chlorine, methoxy, ethoxy, phenylthio, methylsulfonyl or phenylsulfonyl and
B is a radical of the formula –CH=T, where T is

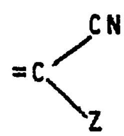

and

Z is hydrogen, cyano, carboxyl, a carboxylic ester group, unsubstituted or substituted carbamoyl, benzimidazolyl, benzoxazolyl or benzothiazolyl.

8. A compound as claimed in claim 1, of the formula

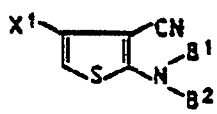

where

$B^1$ is hydrogen, $C_1$–$C_4$-alkyl, $C_2$- or $C_3$-hydroxyalkyl, cyanoethyl, $C_1$–$C_4$-alkoxycarbonylethyl, $C_1$–$C_4$-alkanoyloxyethyl, allyl, benzyl, phenylethyl or cyclohexyl,

$B^2$ is hydrogen, $C_1$–$C_4$-alkyl, $C_2$- or $C_3$-hydroxyalkyl, cyanoethyl, $C_1$–$C_4$-alkoxycarbonylethyl, $C_1$–$C_4$-alkanoyloxyethyl or allyl, or

$B^1$ and $B^2$ together with the nitrogen form pyrrolidino, piperidino, morpholino, piperazino, N-methylpiperazino or N-β-hydroxyethylpiperazino, and

$X^1$ has the meanings stated for claim 2.

9. Use of the compound as claimed in claim 1 as a diazo or coupling component.

10. A process for preparing a compound of the formula

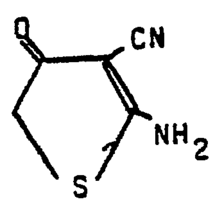

which comprises reacting malonodinitrile with chloroacetyl chloride and reacting the product with a sulfide.

11. A process as claimed in claim 10, wherein the reaction is carried out in a solvent.

12. A process as claimed in claim 10, wherein a base is added.

13. A process for preparing a compound of the formula

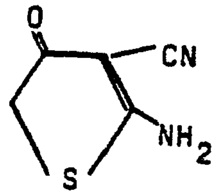

which comprises reacting a 2-cyano-3-alkoxycrotononitrile with a sulfur donor and dealkylating the product.

14. A process as claimed in claim 13, wherein the reaction is carried out in a solvent.

15. A process as claimed in claim 13, wherein a base is added.

**Revendications**

1. Dérivés du thiophène de la formule générale I

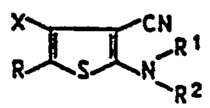

dans laquelle

X représente un atome de fluor, de chlore, de brome, un radical $SO_2Y$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H_5$, $SC_2H_4OH$, $SCH_2COOCH_3$, $SCH_2COOC_2H_5$, $SC_6H_{11}$, $SC_6H_5$ ou $SC_6H_4CH_3$,

Y représente un radical alkyle, alcényle, cycloalkyle, aralkyle, aryle, un atome de chlore, ou un radical amino ou hydroxyle éventuellement substitué,

R représente un atome d'hydrogène, un radical alkyle en $C_1$ à $C_4$ Cl, Br, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$, ou un reste de la formule –CH=T dans laquelle T représente le reste d'un composé à radical méthylène actif ou d'une amine,

$R^1$ représente un atome d'hydrogène, un radical acyle, ou un radical alcényle, cycloalkyle ou alkyle, éventuellement substitué,

$R^2$ représente un atome d'hydrogène ou un radical alcényle ou alkyle, éventuellement substitué,

$R^1$ et $R^2$ représentent, ensemble avec l'atome d'azote, un hétérocycle saturé et

$R^1$ et $R^2$ représentent, ensemble, un reste de la formule

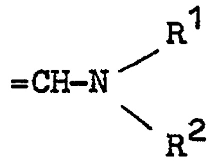

2. Composés suivant la revendication 1, répondant à la formule

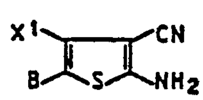

dans laquelle

$X^1$ représente un atome de chlore, un radical hydroxyle, alkylthio ou alcoxy, en $C_1$ à $C_4$, méthylsulfonyle, phénylsulfonyle, hydroxysulfonyle, phénoxy ou phénylthio et

B représente un atome d'hydrogène, un radical alkyle en $C_1$–$C_4$, formyle, acétyle, nitro, hydroxysulfonyle ou cyano.

3. Composé répondant à la formule indiquée dans la revendication 2, caractérisé en ce que

$X^1$ représente un atome de chlore, un radical méthoxy, éthoxy, phénylthio, méthylsulfonyle ou phénylsulfonyle et

B représente le radical formyle.

4. Composé répondant à la formule indiquée dans la revendication 2, caractérisé en ce que

$X^1$ représente un atome de chlore, un radical méthoxy, éthoxy, phénylthio, méthylsulfnoyle ou phénylsulfonyle et

B représente le radical cyano.

5. Composé répondant à la formule indiquée dans la revendication 2, caractérisé en ce que

$X^1$ représente un atome de chlore, un radical méthoxy, éthoxy, phénylthio, méthylsulfonyle ou phénylsulfonyle et

B représente le radical nitro.

6. Composé répondant à la formule indiquée dans la revendication 2, caractérisé en ce que

$X^1$ représente un atome de chlore, un radical méthoxy, éthoxy, phénylthio, méthylsulfonyle ou phénylsulfonyle et

B représente le radical hydroxysulfonyle.

7. Composé répondant à la formule indiquée dans la revendication 2, caractérisé en ce que

$X^1$ représente un atome de chlore, un radical méthoxy, éthoxy, phénylthio, méthylsulfonyle ou phénylsulfonyle et

B représente un reste de la formule –CH=T, dans laquelle T représente un groupe

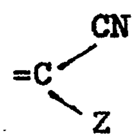

et

Z représente un atome d'hydrogène, un radical cyano, carboxyle, ester carboxylique, benzothiazolyle, benzoxazolyle, benzimidazolyle, carbamoyle, éventuellement substitués.

8. Composé suivant la revendication 1, répondant à la formule

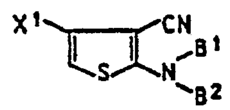

dans laquelle

$B^1$ représente un atome d'hydrogène, un radical alkyle en $C_1$ à $C_4$, hydroxyalkyle en $C_2$ ou $C_3$, cyanéthyle, alcoxy($C_1$–$C_4$)carbonyléthyle, alcanoyl($C_1$–$C_4$)oxéthyle, allyle, benzyle, phényléthyle ou cyclohexyle,

$B^2$ représente un atome d'hydrogène, un radical alkyle en $C_1$ à $C_4$, hydroxyalkyle en $C_2$ ou $C_3$, cyanéthyle, alcoxy($C_1$–$C_4$)carbonyléthyle, alcanoyl($C_1$–$C_4$)oxéthyle, ou allyle ou bien

$B^1$ et $B^2$ forment, ensemble avec l'atome d'azote, un radical pyrrolidino, pipéridino, morpholino, pipérazino, N-méthylpipérazino ou N-β-hydroxyéthylpipérazino et

$X^1$ possède les significations qui lui ont été attribuées dans la revendication 2.

9. Utilisation des composés suivant la revendication 1, à titre de composants diazoïques ou de copulation.

10. Procédé de préparation du composé de la formule

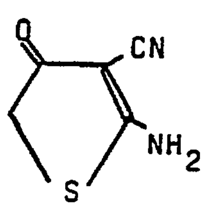

caractérisé en ce que l'on fait réagir le dinitrile de l'acide malonique sur le chlorure de chloracétyle et on poursuit ensuite la réaction avec un sulfure.

11. Procédé suivant la revendication 10, caractérisé en ce que l'on entreprend la réaction dans un solvant.

12. Procédé suivant la revendication 10, caractérisé en ce que l'on ajoute une base.

13. Procédé de préparation du composé de la formule